# EUROPEAN PATENT SPECIFICATION

(11) **EP 3 192 565 B1**
(45) Date of publication and mention of the grant of the patent: **10.11.2021**
(21) Application number: 16150971.6
(22) Date of filing: 12.01.2016
(51) Int. Cl.: A61Q 11/00, A61K 8/66, A61K 8/73

(54) **ORAL CARE COMPOSITION COMPRISING A PROTEIN FOR BALANCING THE BACTERIAL FLORA OF THE MOUTH**
MUNDPFLEGEZUSAMMENSETZUNG ENTHALTEND EIN PROTEIN, UM DIE BAKTERIELLE FLORA DES MUNDES AUSZUGLEICHEN
COMPOSITION DE PROTECTION ORALE CONTENANT UN PROTEINE POUR BALANCER LA FLORE BACTÉRIELLE DE LA BOUCHE

(43) Date of publication of application: 19.07.2017
(73) Proprietor: Unilever IP Holdings B.V., 3013 AL Rotterdam (NL); Unilever Global IP Limited, Wirral, CH62 AZD (GB)
(72) Inventor: ADAMS Suzanne Elizabeth, Merseyside, CH63 3JW (GB); ARNOLD David Stanley, Merseyside, CH63 3JW (GB); BRADING Melanie Gayle, Merseyside, CH63 3JW (GB); MURPHY Barry, Merseyside, CH63 3JW (GB)
(74) Representative: Tansley, Sally Elizabeth

(56) References cited:
- EP-A1- 1 334 710
- CN-A- 104 188 860
- JP-A- 2002 322 088
- US-A- 4 537 764
- None

## Description

### Field of the Invention

The present invention is concerned with oral care compositions. More particularly, the present invention is concerned with oral care compositions containing proteins.

### Background of the Invention

Conventional thinking with respect to toothpastes is that anti-bacterials are needed to destroy the bacteria within the mouth. Conventional thinking with respect to toothpastes is that anti-bacterials are needed to destroy the bacteria within the mouth. Compositions comprising a range of proteins and enzymes that are said to have a negative effect on bacterial are disclosed in JP 2002 322088, EP 1 334 710, US 4 537 764 and CN 104 188 860.

The present application has found that it is beneficial to adjust the balance away from bacteria associated with poor oral health in favour of those associated with good oral health.

The present application relates to compositions that balance the bacterial flora of the mouth.

### Description of the Invention

The present invention relates to a Z gel or paste dentifrice composition comprising protein for use in a method to balance the bacterial flora of the mouth by decreasing the number of anaerobic bacteria without changing the relative abundance of the aerobic bacteria; in which the protein comprises a mixture of lactoferrin, lysozyme, amylglucosidase, glucose oxidase, lactoperoxidase and colostrum.

### Detailed Description of the Invention

The oral microbiome is highly complex and diverse community with over 700 different species having been identified. In general health related bacteria are facultative anaerobic or aerobic bacteria (e.g genus Neisseria) and bacteria related to gum disease are anaerobic bacteria (e.g genus Treponema). There is therefore a need for the promotion of aerobic bacteria in the mouth and a decrease in the number of anaerobic bacteria.

The present application has found that compositions comprising a mixture of lactoferrin, lysozyme, amylglucosidase, glucose oxidase, lactoperoxidase and colostrum can be used to decrease the number of anaerobic bacteria without changing the relative abundance of the aerobic bacteria.

The level of lactoferrin in the invention is preferably from 0.01 wt% to 0.1 wt%, more preferably from 0.005 wt% to 0.05 wt%. Other proteins preferably lysozymes, and colostrum may be present in the invention. Preferably these proteins are present at total levels from 0.001 to 0.5 wt% of the total composition, preferably from 0.005 to 0.1 wt% of the total composition.

The compositions of the invention comprise enzymes.

Preferably the total level of enzyme is from 0.01 wt% to 2wt% of the total composition. More preferably from 0.1 wt% to 1 wt% of the total composition.

Preferably the total level of glycoside hydrolase in the composition is from 0.05 to 1 wt% of the total composition.

Preferably the total level of oxoreductase acting on OH groups of donors is from 0.05 to 1 wt% of the total composition.

Preferably the total level of hemeperoxidase is from 0.0005 to 0.01 wt% of the total composition.

Preferably the weight ratio of glycoside hydrolase to other enzymes in the composition is greater than 1:1, more preferably greater than 3:2.

Furthermore, the oral care composition will usually contain a surfactant in an amount of from 0.2 to 5% by weight based on the total weight of the dentifrice. Preferred are nonionic surfactants such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Particularly preferred are polyethylene glycol ethers of fatty alcohols in particular polyethylene glycol ethers having from 20 to 40 ethylene oxide groups per unit. An example of such a suitable surfactant includes the group of surfactants known as Steareth surfactants such as Steareth 30. Preferably the level of non-ionic surfactant is from 0.5 to 7 wt% of the total composition, more preferably from 1 to 5 wt% of the total composition

Although other surfactants may be present it is preferred if they are limited to levels below 0.5 wt% of the total composition preferably less than 0.1 wt% of the total composition.

The oral care composition, especially in the cases of dentifrices, dentifrice may also contain structurants and agents such as binders and thickening agents. These structurants will generally be present in an amount of from 0.1 to 1% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth). Natural gum based thickeners, in particular carrageenan are particularly preferred.

Examples of suitable product forms for compositions of the invention include dentifrices, mouthwashes, mouthsprays and liquid formulations.

Preferred product forms for compositions of the invention are those which are suitable for brushing and/or rinsing the surfaces of the oral cavity. Dentifrices/toothpastes are preferred.

In such preferred product forms, the amount of water and/or polyhydric alcohol will generally be at least 10 percent, preferably at least 30 percent, more preferably at least 50 percent by total weight water and/or polyhydric alcohol based on the total weight of the composition. Preferably the level of water and/or polyhydric alcohol will be less than 90 wt% of the total composition, more preferably less than 85 wt%.

The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. The dentifrice according to the present invention is in the form of a paste or a gel (or a combination thereof).

A dentifrice composition according to the invention will usually contain, as the aqueous continuous phase, a mixture of water and polyhydric alcohol in various relative amounts, with the amount of water generally ranging from 10 to 50% by weight (based on the total weight of the dentifrice) and the amount of polyhydric alcohol generally ranging from 20 to 60% by weight (based on the total weight of the dentifrice).

Typical polyhydric alcohols for use in the oral care composition, particularly a dentifrice composition according to the invention include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof. Glycerol and sorbitol are preferred, sorbitol being particularly preferred.

The dentifrice compositions comprise abrasive materials. Abrasive materials will generally be present in an amount of from 0.5 to 75%, preferably 3 to 60 by weight based on the total weight of the dentifrice. Suitable abrasive cleaning agents include silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof. Silicas are particularly preferred.

Compositions of the invention may also comprise thickeners such as finely divided silicas, hectorites, calcium carbonate, colloidal magnesium aluminium silicates and mixtures thereof.

Compositions of the invention may comprise a zinc ion. Preferably the sources of zinc ions are zinc chloride, zinc acetate, zinc gluconate, zinc sulphate, zinc fluoride, zinc citrate, zinc lactate, zinc oxide, zinc monoglycerolate, zinc tartrate, zinc pyrophosphate zinc maleate and mixtures thereof.

Compositions of the present invention may also contain further optional ingredients customary in the art such as fluoride ion sources, anticalculus agents, buffers, flavouring agents, sweetening agents, colouring agents, opacifying agents, preservatives, antisensitivity agents, bleaching and antimicrobial agents.

To clean the surfaces of the oral cavity, the composition according to the invention is topically applied to the oral cavity surfaces to be cleaned.

Preferably the composition according to the invention is topically applied to the oral cavity surfaces to be cleaned and then agitated by brushing and/or rinsing.

The invention will now be illustrated by the following non-limiting Examples:

### Examples

A double-blind, randomised, parallel study, was conducted to collect plaque samples by an independent research organization. Samples from 102 subjects completed the microbiomics analysis, 52 using Example 1 and 50 using standard silica SLS toothpaste - Example A. After recruitment, subjects used a silica SLS (Example A) toothpaste for up to four weeks, and then subjects used their allocated test product at home, twice a day for 14 weeks. Samples of plaque were collected at baseline and at the end of the 14 week brushing period for microbiomic analysis.

### Example 1

| **Material** | **Amount %W/W** |
|---|---|
| Sorbitol | 28.5 |
| Zinc Gluconate | 0.3 |
| Sodium Fluoride | 0.3 |
| Citric Acid | 0.3 |
| Disodium Phosphate | 0.5 |
| Sodium Caseinate? | 0.01 |
| Titanium dioxide | 0.5 |
| Zeodent 113* | 16.0 |
| Tixosil 43** | 4.8 |
| Carrageenan | 0.7 |
| Glycerin | 5.0 |
| Xanthum Gum | 1.0 |
| Steareth 30 | 3.0 |
| Amylglucosidase | 0.3 |
| Lactoperoxidase | 0.002 |
| Lysozyme (22%)*** | 0.05 |
| Glucose Oxidase | 0.14 |
| Lactoferrin | 0.01 |
| Colostrum | 0.01 |
| Water and minors | to100.0 |

| | |
|---|---|
| * Precipitated silica ** Thickening silica *** 0.0495% lysozyme liquid at 22% lysozyme (containing 1450ppm F as NaF) - orphaned! | |

### Example A

| **Material** | **Amount (%W/W)** |
|---|---|
| Sorbitol 70/70 | 45 |
| PEG-32 | 5 |
| Monosodium Phosphate | 0.1 |
| Sodium Fluoride | 0.32 |
| Titanium Dioxide | 1 |
| Tixosil 43* | 7.5 |
| Sorbosil AC77** | 10 |
| Celulose Gum | 0.63 |
| Sodium Lauryl Sulphate | 1.5 |
| Water and minors | To 100 |

| | |
|---|---|
| Silica SLS Toothpaste (containing 1450ppm F as NaF) * hydrated thickening silica ** hydrated silica | |

### Microbiomics analysis:

Plaque samples were processed and sequence data was generated based on the V4-V6 region of the 16S rRNA gene using the latest Illumina sequencing techniques. Species level identification was carried out using the Human Oral Microbiome Database (HOMD) at the Forsyth Institute, Boston using their bioinformatic pipeline. The data was analysed using a range of recognised statistical tests designed for microbiomics.

### Results:

Following statistical analysis (PERMANOVA) the following changes were identified:
- No significant difference was observed between the population for Example 1 and Example A at baseline (p=0.26).
- There was a significant shift in population for Example 1 from baseline to 14 weeks (p=0.04).
- In contrast, for Example A, no change in population from baseline to 14 weeks (p=0.99).
- A comparison of the populations at the 14 week time point showed a significant difference between the two products (p=0.01).

Detailed analysis of the species over time showed a significant increase in health related aerobic bacteria (belonging to the genus Neisseria) were observed with a concomitant decrease in disease related anaerobic bacteria (belonging to the genus Treponema) in Example 1. No such overall changes were observed for Example A.

## Claims

1. A gel or paste dentifrice composition comprising protein for use in a method to balance the bacterial flora of the mouth by decreasing the number of anaerobic bacteria without changing the relative abundance of the aerobic bacteria; in which the protein comprises a mixture of lactoferrin, lysozyme amylglucosidase, glucose oxidase lactoperoxidase and colostrum.

2. A composition according to any preceding claim in which the composition further comprises a non-ionic surfactant.

3. A composition according to claim 2 in which the non-ionic surfactant is a polyethylene glycol ethers of a fatty alcohol.

4. A composition according to claim 3 in which the non-ionic surfactant is steareth 30.

5. A composition according to any preceding claim in which the composition comprises a carrageenan based thickener.

6. A composition according to any preceding claim in which the composition further comprises a zinc ion.

## Patentansprüche

1. Gel- oder pastenförmige Zahnputzmittelzusammensetzung, umfassend Protein zur Verwendung in einem Verfahren zum Ausgleichen der Bakterienflora des Mundes durch Senken der Zahl anaerober Bakterien, ohne die relative Häufigkeit der aeroben Bakterien zu ändern, wobei das Protein eine Mischung von Lactoferrin, Lysozym Amylglucosidase, Glucoseoxidase, Lactoperoxidase und Kolostrum umfasst.

2. Zusammensetzung nach irgendeinem vorhergehenden Anspruch, wobei die Zusammensetzung ferner ein nichtionisches Tensid umfasst.

3. Zusammensetzung nach Anspruch 2, wobei das nichtionische Tensid ein Polyethylenglycolether eines Fettalkohols ist.

4. Zusammensetzung nach Anspruch 3, wobei das nichtionische Tensid Steareth 30 ist.

5. Zusammensetzung nach irgendeinem vorhergehenden Anspruch, wobei die Zusammensetzung ein Verdickungsmittel auf Carrageenan-Basis umfasst.

6. Zusammensetzung nach irgendeinem vorhergehenden Anspruch, wobei die Zusammensetzung ferner ein Zinkion umfasst.

## Revendications

1. Composition de dentifrice en gel ou pâte comprenant une protéine pour une utilisation dans un procédé pour équilibrer la flore bactérienne de la bouche en abaissant le nombre de bactéries anaérobies sans modifier l'abondance relative des bactéries aérobies ; dans laquelle la protéine comprend un mélange de lactoferrine, lysozyme amylglucosidase, glucose oxydase lactopéroxydase et colostrum.

2. Composition selon l'une quelconque des revendications précédentes, dans laquelle la composition comprend de plus un tensioactif non-ionique.

3. Composition selon la revendication 2, dans laquelle le tensioactif non-ionique est un polyéthylène glycol éther d'un alcool gras.

4. Composition selon la revendication 3, dans laquelle le tensioactif non-ionique est steareth 30.

5. Composition selon l'une quelconque des revendications précédentes, dans laquelle la composition comprend un épaississant à base de carraghénane.

6. Composition selon l'une quelconque des revendications précédentes, dans laquelle la composition comprend de plus un ion zinc.
